# DEMANDE DE BREVET EUROPEEN

(11) **EP 0 709 651 A1**
(43) Date de publication de la demande: **01.05.1996**
(21) Numéro de dépôt: 95490032.0
(22) Date de dépôt: 24.10.1995
(51) Int. Cl.: G01B 11/00, G01N 33/36, G01N 33/34, G01N 21/89, G06T 7/00

(54) **Procédé de contrôle de l'emplacement respectif de motifs sur une nappe de matériau**

(30) Priorité: 24.10.1994 FR 9412924
(71) Demandeur: S.A.R.L.: 3,14 INGENIERIE, F-62100 Calais (FR)
(72) Inventeur: Laforge, Alain, F-62100 Calais, (Pas-de-Calais) (FR); Hochard, Jean-Pierre, F-62100 Calais, (Pas-de-Calais) (FR)
(74) Mandataire: Duthoit, Michel

(57) **Abrégé**

La présente invention concerne un procédé de contrôle de l'emplacement respectif de motifs (3) disposés selon une pseudo-période δ₀ pré-établie sur une nappe (1) de matériau tel que, notamment, du tissu, du papier ou autre, selon lequel :
- on prévoit un champ d'investigation (4) incluant au moins deux motifs (3) complets,
- on prévoit une fenêtre de référence (5) incluse dans ledit champ d'investigation (4),
- on détecte, à l'intérieur dudit champ d'investigation (4), une fenêtre (6) optiquement identique à ladite fenêtre de référence (5) pour déterminer une pseudo-période δ réelle

La présente invention concerne également un dispositif pour la mise en oeuvre de ce procédé ainsi que ses applications, notamment, à une technique de préformage de tissu, dentelle ou autre.

## Description

La présente invention concerne un procédé de contrôle de l'emplacement respectif de motifs sur une nappe de matériau tel que notamment du tissu, du papier ou autre, ainsi qu'un dispositif pour la mise en oeuvre d'un tel procédé et ses applications, notamment à une technique de préformage de tissu, dentelle ou autre.

Bien que plus particulièrement destinée à des matériaux textiles tels que, par exemple, du tissu, de la dentelle ou autre, la présente invention n'est toutefois pas limitée à de telles utilisations et elle trouvera ses applications dans tous les secteurs de l'activité économique dans lesquels on est amené à rencontrer une nappe de matière présentant des motifs.

Lors de la fabrication et/ou l'utilisation d'une nappe de matériau présentant des motifs, il est souvent nécessaire de contrôler avec précision le positionnement respectif de ces derniers.

Dans le cas de l'utilisation de plusieurs lés, par exemple, une répartition régulière des motifs est exigée, notamment afin de permettre des raccords et/ou reprises qui conservent la périodicité des motifs d'un lé à l'autre. Cela est notamment le cas dans l'industrie de la confection utilisant de la dentelle. En effet, pour ce matériau, le niveau de détails des motifs implique l'utilisation d'un tissu sur lequel leur positionnement soit particulièrement juste et régulier.

En ce qui concerne le préformage de tissu, dentelle ou autre, on connaît actuellement, pour contrôler l'emplacement respectif de motifs sur la nappe, des méthodes manuelles. Bien que satisfaisantes dans certains cas, celles-ci présentent néanmoins l'inconvénient d'être lentes, peu précises et particulièrement astreignantes.

On connaît également, dans l'industrie textile, différents dispositifs permettant, grâce à des moyens de traitement numériques d'images, de contrôler la régularité d'un tissu présentant des caractéristiques périodiques.

Toutefois, leur objet est de repérér des défauts dans la structure du tissu et non de déterminer l'emplacement respectif de motifs à sa surface. De plus, les méthodes qu'ils mettent en oeuvre sont particulièrement complexes et longues.

Elles nécessitent ainsi, au niveau des moyens de traitement numériques, de grosses quantités de mémoire. Elles obligent également à maintenir le tissu immobile lors du contrôle.

Le but de la présente invention est de proposer un procédé de contrôle de l'emplacement respectif de motifs sur une nappe de matériau qui pallie les inconvénients précités et permette de s'affranchir d'une intervention humaine tout en augmentant la rapidité et la finesse de la mesure.

Un autre but de la présente invention est de proposer un procédé de contrôle de l'emplacement respectif de motifs sur une nappe de matériau qui permette de laisser défiler ladite nappe, notamment en continu, lors du contrôle.

Un autre but de la présente invention est de proposer un procédé de contrôle de l'emplacement respectif de motifs sur une nappe de matériau ainsi qu'un dispositif pour sa mise en oeuvre qui permettent de limiter la surface des zones traitées et diminuent ainsi le temps de contrôle et le coût du matériel à mettre en oeuvre.

Un autre but de la présente invention est de proposer un procédé de contrôle de l'emplacement respectif de motifs sur une nappe de matériau qui permette de suivre le droit dessin.

Un autre but de la présente invention est de proposer un procédé de contrôle de l'emplacement respectif de motifs sur une nappe de matériau ainsi qu'un dispositif pour sa mise en oeuvre et ses applications, notamment à une technique de préformage de tissu, dentelle ou autre, qui permettent d'asservir le préformage en fonction des résultats du contrôle pour corriger d'éventuelles dérives et assurer ainsi une répartition régulière des motifs sur le tissu.

Un avantage de la présente invention est d'éviter à un opérateur manuel un travail fastidieux et peu gratifiant.

D'autres buts et avantages de la présente invention apparaîtront au cours de la description qui va suivre qui n'est donnée qu'à titre indicatif et qui n'a pas pour but de la limiter.

La présente invention concerne un procédé de contrôle de l'emplacement respectif de motifs disposés selon une pseudo-période δ₀ pré-établie sur une nappe de matériau tel que, notamment, du tissu, du papier ou autre, selon lequel :
- on prévoit un champ d'investigation incluant au moins deux motifs complets,
- on prévoit une fenêtre de référence incluse dans ledit champ d'investigation,
- on détecte, à l'intérieur dudit champ d'investigation, une fenêtre optiquement identique à ladite fenêtre de référence pour déterminer une pseudo-période à réelle.

La présente invention concerne également une application d'un tel procédé, dans lequel on contrôle l'emplacement respectif de motifs disposés selon une pseudo-période δ₀ pré-établie sur une nappe de matériau tel que, notamment, du tissu, de la dentelle ou autre, au suivi du droit dessin des motifs disposés sur ladite nappe par mesures successives de l'emplacement respectif desdits motifs.

La présente invention concerne également une application d'un tel procédé, dans lequel on contrôle l'emplacement respectif de motifs disposés selon une pseudo-période δ₀ pré-établie sur une nappe de matériau tel que, notamment du tissu, de la dentelle ou autre, à une technique de préformage de ladite nappe avec asservissement du positionnement desdits motifs selon les résultats dudit contrôle.

La présente invention concerne également une application d'un tel procédé, dans lequel on contrôle l'emplacement respectif de motifs disposés selon une pseudo-période δ₀ pré-établie sur une nappe de matériau tel que, notamment, du tissu, de la dentelle, du tricot ou autre, à la régulation de la tension de ladite nappe à la sortie de machines à tisser, à fabriquer la dentelle, à tricoter ou équivalent, de manière à produire une nappe présentant des motifs répartis régulièrement.

La présente invention concerne également un dispositif pour la mise en oeuvre du procédé défini plus haut dans lequel on contrôle l'emplacement respectif de motifs disposés selon une pseudo-période ôₒ pré-établie sur une nappe de matériau, tel que, notamment, du tissu, du papier ou autre, notamment destiné au préformage de tissu présentant des motifs, caractérisé par le fait qu'il est constitué d'une caméra et de moyens de traitement de l'image.

L'invention sera mieux comprise si l'on se réfère à la description suivante ainsi qu'aux dessins en annexe qui en font partie intégrante.

La figure 1 montre en vue de dessus, une nappe de matériau , sur laquelle on contrôle, conformément à l'invention, l'emplacement respectif de motifs.

La figure 2 illustre la mise en oeuvre d'une étape d'un mode particulier du procédé de contrôle conforme à l'invention.

Les figures 3a et 3b servent à illustrer un exemple d'application du procédé conforme à l'invention.

La figure 3c illustre un mode de visualisation des résultats obtenus dans le cadre de l'application illustrée aux figures 3a et 3b précédentes.

La figure 4 est une vue schématique d'une application du procédé de contrôle, conforme à l'invention, à une technique de préformage de tissu présentant des motifs.

La présente invention concerne un procédé de contrôle de l'emplacement respectif de motifs sur une nappe de matériau ainsi qu'un dispositif pour sa mise en oeuvre et ses applications notamment à une technique de préformage de tissu, dentelle ou autre.

Bien que plus particulièrement destinée à des matériaux textiles tels que, par exemple, du tissu, de la dentelle ou autre, la présente invention n'en est pas moins limitée à de telles applications et elle trouvera son utilisation dans tous les domaines de l'activité économique dans lesquels on est amené à rencontrer une nappe de matériau sur laquelle sont disposés des motifs.

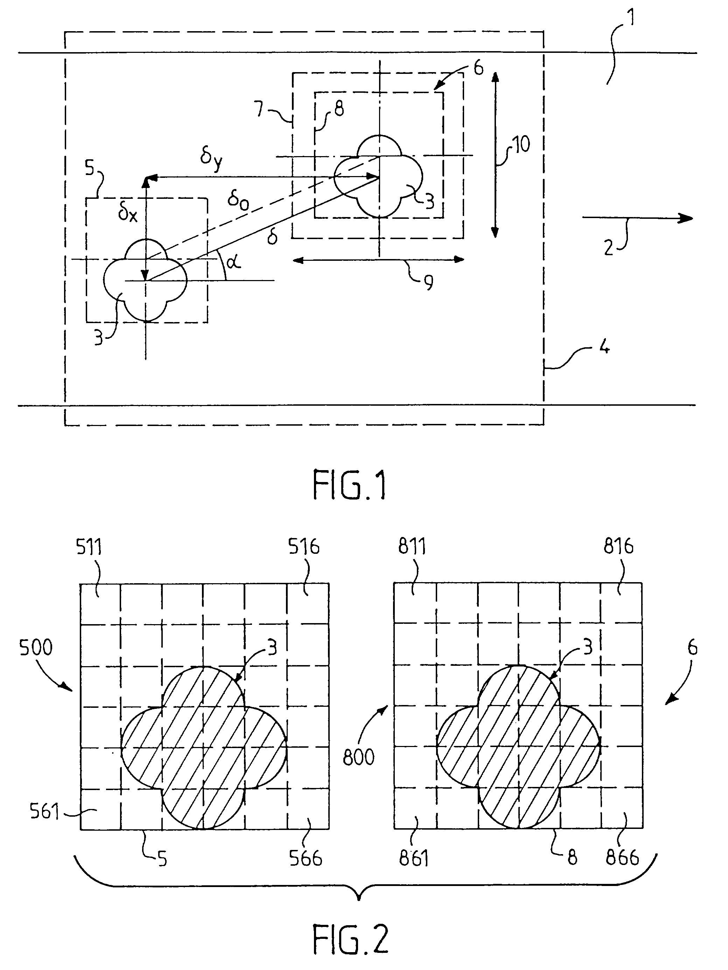

A la figure 1, on remarque une nappe 1 de matériau tel que, notamment, du tissu, du papier ou autre.

Cette nappe 1 est éventuellement en défilement continu dans le sens de la flèche repérée 2.

Elle présente, sur au moins l'une de ses faces, des motifs 3. Il peut s'agir de tout type de motifs tels que, par exemple, des motifs géométriques, figuratifs ou autres.

Ils sont disposés sur la nappe 1 selon une pseudo-période δ₀ pré-établie. Ainsi, les motifs sont distribués sur la surface de la nappe 1 suivant une répartition régulière, la pseudo-période δ₀, permettant, a priori, de passer d'un motif 3 à l'autre, devant être entendu comme une pseudo-période spatiale, éventuellement repérée par ses coordonnées transversales et longitudinales, à savoir respectivement δₓ₀ et δ_{y0}

Selon le procédé, conforme à l'invention, de contrôle de l'emplacement respectif des motifs 3 sur la nappe de matériau 2, on prévoit un champ d'investigation 4 incluant au moins deux motifs 3 complets, c'est-à-dire deux motifs 3 dont les contours extérieurs sont intégralement compris dans ledit champ d'investigation 4.

On prévoit, également, une fenêtre de référence 5 incluse dans ledit champ d'investigation 4. Elle encadre au moins une partie caractéristique d'un des motifs 3. Elle comprend ainsi une région du motif qui se dissocie du fond de la nappe de matière 1.

Toujours selon l'invention, on détecte, à l'intérieur dudit champ d'investigation 4, une fenêtre 6 optiquement identique à ladite fenêtre de référence 5 pour déterminer une pseudo-période à réelle.

Selon un mode particulier de mise en oeuvre du procédé de contrôle conforme à l'invention, on prévoit, pour détecter la fenêtre optiquement identique 6, une plage de recherche 7 au voisinage de la pseudo-période et/ou d'un multiple de la pseudo-période δ₀.

En effet, selon un premier mode de réalisation, le champ d'investigation 4 peut inclure plus de deux motifs 3 complets. Suivant ce mode de réalisation, si la plage de recherche 7 est prévue au voisinage d'un multiple k de la pseudo-période δ₀, il faudra, pour déterminer la pseudo-période à réelle, diviser les résultats obtenus par la suite par k.

Suivant le mode de réalisation représenté à la figure 1, le champ d'investigation 4 inclut au plus deux motifs 3 complets et la plage de recherche 7 est prévue au voisinage de la pseudo-période δ₀.

Suivant le mode de mise en oeuvre particulier du procédé conforme à l'invention évoqué plus haut, on prévoit, également, une fenêtre de recherche 8, par exemple, de dimension identique à la fenêtre de référence 5.

A ce sujet, les fenêtres de référence 5 et de recherche 8 présentent un contour, notamment, quadrangulaire tel que par exemple, un carré, un rectangle ou autre. On pourra envisager également, par exemple, un contour circulaire et, plus généralement, tout type de contour apte à permettre l'établissement de l'identicité optique des deux fenêtres.

Suivant un autre mode de réalisation, les fenêtres de référence 5 et de recherche 8, présentent des tailles différentes. La recherche d'identicité optique s'effectue alors sur la surface de la plus faible des deux fenêtres. Celle-ci devra, comme évoqué plus haut, inclure au moins une partie caractéristique du motif 3.

Toujours suivant la figure 1, on déplace pas à pas la fenêtre de recherche 8 dans le plan de la plage de recherche 7 et on compare, à chaque pas, l'image de la fenêtre de reférence 5 et de ladite fenêtre de recherche 8.

Le déplacement pas à pas de la fenêtre de recherche 8 s'effectue selon les flèches longitudinales repérées 9 et transversales repérées 10. On balaie ainsi la surface de la plage de recherche 7. Le balayage peut être, par exemple, transversal, longitudinal, ou suivant un déplacement en spirale, notamment autour de la pseudo-période δ₀ pré-établie. Un déplacement aléatoire peut également être envisagé.

On repère ainsi un emplacement de la fenêtre de recherche 8 pour lequel il existe une coïncidence entre l'image de ladite fenêtre de recherche 8 et celle de la fenêtre de référence 5.

Suivant une variante de réalisation, dans le cas de figures où le champ d'investigation 4 inclut plus de deux motifs 3, on peut prévoir un nombre de fenêtres de recherche 8 correspondant à toute ou partie du nombre de motifs 3 présents dans le champ d'investigation 4.

La figure 2 détaille comment on compare l'image de la fenêtre de recherche 8 et de la fenêtre de référence 5 selon un mode de réalisation particulier de l'invention.

Pour cela, on réalise, notamment, un découpage virtuel en sous-fenêtre 500, 800, de chacune des deux fenêtres 5, 8. On obtient ainsi, par exemple, des sous-fenêtres, 511, 811, qui occupent le même emplacement relatif à l'intérieur de chacune des fenêtres 5, 8. Ce découpage virtuel constitue, notamment, une représentation matricielle de chacune des deux fenêtres 5, 8.

Le contour des sous-fenêtres se présente, par exemple, sous une forme quadrangulaire telle qu'un rectangle, un carré ou autre. D'autres contours, par exemple circulaires peuvent être envisagés.

Selon ce mode de réalisation de l'invention, on mesure la luminosité de chacune des sous-fenêtres des deux fenêtres 5, 8. On compare alors deux à deux, pour chaque sous-fenêtre correspondante, par exemple les sous-fenêtres 511, 811, des deux fenêtres 5, 8, la luminosité. Pour cela, il faudra veiller, pour assurer la pertinence des résultats, à éclairer de manière uniforme le champ d'investigation 4 afin d'éviter tout bruit et interférence.

Enfin, toujours pour comparer l'image de la fenêtre de recherche 8 et de la fenêtre de référence 5, on additionne entre elles, selon l'invention, pour chaque couple tel que, par exemple, 511, 811 de sous-fenêtres, une valeur absolue de la différence de leur luminosité afin d'obtenir un niveau de différence entre l'aspect optique des deux fenêtres.

On repère ainsi l'emplacement pour lequel il existe une coïncidence entre l'image de la fenêtre de recherche 8 et celle de la fenêtre de référence 5 en déterminant la fenêtre de recherche 8 pour laquelle le niveau de différence est minimum. La fenêtre de recherche 8 peut alors être considérée comme constituant la fenêtre 6 optiquement identique à la fenêtre de référence 5.

On pourra prévoir un seuil de validation auquel le minimum de niveau de différence devra être inférieur pour que le résultat ci-dessus soit validé. Une durée maximale pour la recherche de la fenêtre optiquement identique 6 peut également être envisagée. Dans de tels cas, l'impossibilité, pour le procédé de contrôle conforme à l'invention à isoler une fenêtre 6 optiquement identique à la fenêtre de référence 5 est, par exemple, signalée à un opérateur.

Selon un mode particulier de mise en oeuvre du procédé conforme à l'invention, les sous-fenêtres des fenêtres de référence 5, 8 sont constituées des pixels d'une caméra. Par pixels, il faut entendre plus petites surfaces accessibles au niveau de définition de ladite caméra.

Selon un tel mode de mise en oeuvre, pour connaître la luminosité de chacune des sous-fenêtres, on mesure, suivant des procédés connus de l'homme de métier, le niveau de gris et/ou le niveau de couleur du pixel correspondant.

Selon un mode particulier de réalisation de l'invention, on calcule et on affiche la pseudo-période réelle δ. Pour cela, comme représenté à la figure 1, on peut utiliser le fait que la pseudo-période est repérée, par exemple, par sa norme et par un angle de décalage par rapport à la direction longitudinale α et/ou par ses coordonnées transversales et longitudinales δₓ et δ_{y}.

Sur cette figure, on constate également que la pseudo-période entre deux motifs correspond à la pseudo-période entre la fenêtre de référence 5 et sa fenêtre optiquement identique 6, ceci quelle que soit la place du motif à l'intérieur de la fenêtre. On peut ainsi effectuer les calculs, par exemple, à partir du centre desdites fenêtres.

En cas de défilement de la nappe de matériau 1, il est à noter que ce mouvement doit être compensé lors de la détection de la fenêtre 6 optiquement identique.

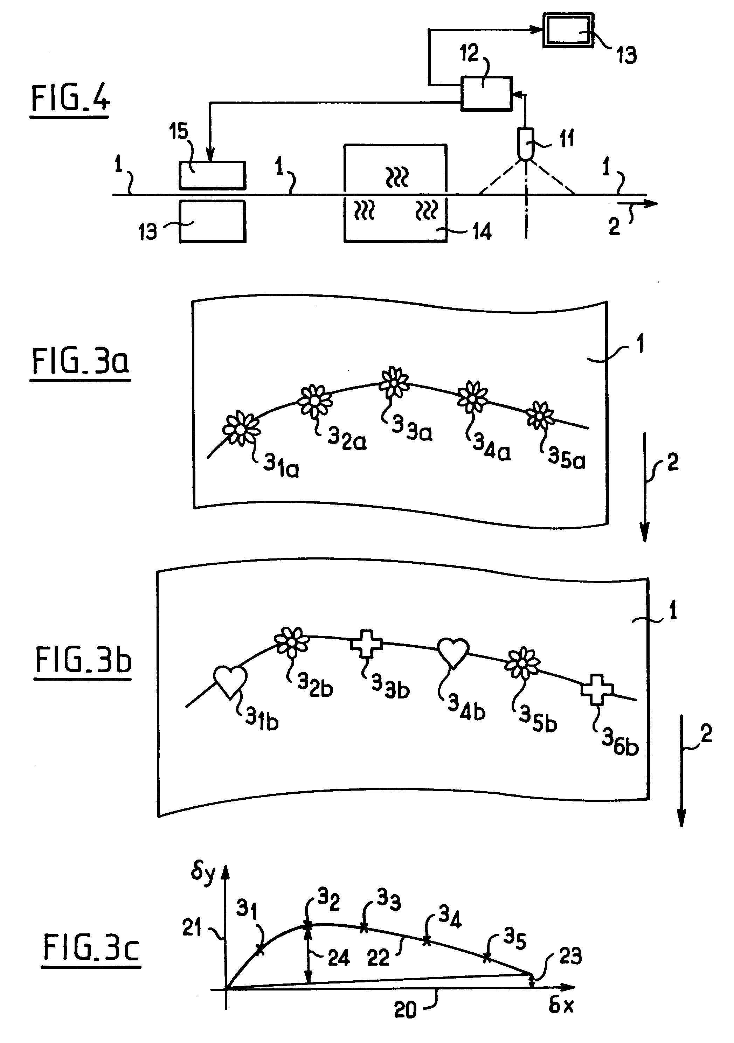

Comme illustrée par les figures 3a et 3b, la présente invention concerne également une application du procédé tel que décrit ci-dessus dans lequel on contrôle l'emplacement respectif de motifs 3 disposés selon une pseudo-période δ₀ pré-établie sur une nappe 1 de matériau tel que, du tissu, de la dentelle ou autre, au suivi du droit dessin des motifs 3 disposés sur ladite nappe 1.

Ledit droit dessin est constitué, par exemple, par la valeur de la pente p = (δ_{y}/δₓ) x 100 entre deux motifs 3 identiques.

Selon l'exemple de mise en oeuvre illustré à la figure 3a d'une telle application, dans le cas où les motifs 3 sont identiques entre eux, on mesure successivement, selon l'invention, l'emplacement respectif des motifs 3₁ₐ et 3₂ₐ, 3₁ₐ et 3₃ₐ, 3₁ₐ et 3₄ₐ, 3₁ₐ et 3₅ₐ. On détermine alors, à l'intérieur de chaque couple, la pente entre les motifs.

La suite numérique ainsi obtenue permet de suivre le droit dessin tout le long de la direction choisie.

Dans le cas où, comme à la figure 3b, les motifs ne sont pas tous identiques, on mesure successivement selon l'invention, l'emplacement respectif des motifs identiques deux à deux. Selon l'exemple illustré, il s'agit, notamment, des motifs 3_{1b} et 3_{4b}, 3_{2b} et 3_{5b}, 3_{3b} et 3_{6b}. On détermine alors, comme dans le cas précédent, l'évolution du droit dessin le long de la direction choisie.

A ce sujet, ladite direction peut être, par exemple, comme représentée aux figures 3a et 3b, orthogonale à la longueur de la nappe 1 ou selon d'autres modes d'exploitation, obliques, en zig-zag ou autres.

A la figure 3c, toujours selon l'invention, on visualise l'évolution du droit dessin en positionnant les motifs 3 dont l'emplacement a été contrôlé selon le procédé conforme à l'invention dans un système de coordonnées formées par l'axe des abscisses 20 et l'axe des ordonnées 21. On extrapole alors l'allure de la courbe 22 représentant l'emplacement des motifs 3. Ladite courbe permet ainsi de suivre, en la visualisant, l'évolution du droit dessin.

Grâce à ladite courbe 22, on peut éventuellement mesurer selon l'invention l'inclinaison de la nappe 1, illustrée par la flèche repérée 23, et/ou le cintrage de ladite nappe 1, illustrée par la flèche repérée 24.

Ces deux paramètres permettent, par exemple, de déterminer si une nappe 1 présentant des motifs 3 a été correctement fabriquée. Ils peuvent également servir, notamment, de paramètres de régulation si l'évolution constatée du droit dessin fait apparaître une dérive.

Ainsi, si dans le cas des figures 3a et 3b, on souhaite en fait obtenir un droit dessin perpendiculaire à la longueur de la nappe 1, on régulera, comme évoqué plus loin, le dispositif de production de manière à obtenir une inclinaison nulle et un cintrage nul.

La présente invention concerne également une application du procédé tel que décrit ci-dessus dans lequel on contrôle l'emplacement respectif de motifs 3 disposés selon une pseudo-période δ₀ pré-établie sur une nappe 1 de matériau tel que, du tissu, de la dentelle ou autre, à une technique de préformage de ladite nappe avec asservissement du positionnement desdits motifs 3 selon les résultats dudit contrôle. Selon des techniques connues de l'homme de métier, le préformage du tissu est, par exemple, constitué d'une première étape dans laquelle on étire le tissu puis d'une seconde étape dans laquelle on le chauffe.

Selon l'invention, on contrôle, par exemple, après le chauffage, l'emplacement respectif des motifs 3 sur le tissu 1. En cas de dérive entre la pseudo-période δ₀ pré-établie et la pseudo-période δ réelle mesurée et/ou en cas de dérive du droit dessin, on modifie, par exemple, l'étirage du tissu. On constitue ainsi un asservissement du positionnement des motifs 3 selon les résultats du contrôle du raccord ou pseudo-période δ.

Une seconde application du procédé, décrit ci-dessus, de contrôle de l'emplacement respectif de motifs sur une nappe de matière, éventuellement en défilement, concerne la régulation de la tension de ladite nappe à la sortie de machines à tisser, à fabriquer la dentelle, à tricoter ou équivalent, de manière à produire une nappe présentant des motifs répartis régulièrement.

Lors de la production de nappes notamment textiles, l'existence de rétreints, c'est-à-dire de retraits de tissu dus à la surtension appliquée au fil lors du tissage, du tricotage ou équivalent impose de contrôler la tension du tissu à la sortie des machines à tisser, à fabriquer la dentelle, à tricoter ou équivalent.

De telles méthodes sont connues de l'homme de l'art. On peut, par exemple, entraîner le tissu par un moteur à courant continu dont on contrôle le couple.

Néanmoins, actuellement, le réglage de ladite tension de sortie est réalisé a priori, ce qui entraîne souvent, dans le cas de tissus présentant des motifs, une distorsion de ces derniers.

Selon l'invention, on régule, grâce à un asservissement, la tension de sortie de la machine en fonction des mesures délivrées par le procédé de contrôle de l'emplacement respectif de motifs décrits ci-dessus. Ainsi, par exemple, si des motifs sont trop rapprochés les uns des autres, on modifiera la tension de sortie afin de les espacer.

Une telle application est également envisageable dans l'industrie de la bonneterie où le motif est constitué, par exemple, par la maille du tricot. Pour cela, il suffit de définir la taille des fenêtres de référence et de recherche ainsi que la pseudo-période utilisée dans le procédé décrit ci-dessus en fonction des dimensions de la maille.

Comme représentée à la figure 4, la présente invention concerne également un dispositif pour la mise en oeuvre du procédé décrit ci-dessus, dans lequel on contrôle l'emplacement respectif de motifs 3 disposés selon une pseudo-période δ₀ pré-établie sur une nappe de matériau 1 tel que notamment du tissu, du papier ou autre. Ce dispositif est notamment destiné au préformage de tissu 1 présentant des motifs 3.

Selon l'invention, il est constitué, par exemple, d'une caméra 11 et de moyens de traitement d'images 12. Il est muni, entre autres, de moyens 13 de calcul et d'affichage des résultats.

Selon un mode particulier de réalisation de ce dispositif, le champ d'investigation 4 correspond, notamment, au champ de la caméra. Les moyens 12 de traitement d'images sont munis de moyens de paramétrage du contrôle de l'emplacement des motifs 3 en fonction, notamment, de la pseudo-période pré-établie δ₀ et de la taille des motifs 3.

En cas de défilement de la nappe de matériau 1, le dispositif conforme à l'invention comprend, par exemple, des moyens de compensation dudit défilement lors du contrôle de l'emplacement des motifs 3. Il peut s'agir, par exemple, de moyens de translation de la caméra 11 et/ou de moyens intégrés aux moyens 12 de traitement d'images.

A titre d'exemple, la nappe de matériau 1 utilisée présente une largeur de l'ordre de 5 m.

L'invention concerne également un dispositif de préformage de tissu muni du dispositif de contrôle de l'emplacement respectif de motifs 3 sur une nappe de matériau 1 décrit ci-dessus. Si l'on se reporte de nouveau à la figure 4, on constate que ce dispositif de préformage de tissu 1 comprend, par exemple, des moyens 13 pour étirer le tissu et un four 14.

Selon l'invention, il comprend également notamment, des moyens 15 pour asservir le positionnement des motifs 3 selon les résultats du contrôle. Il peut s'agir, par exemple, d'un automate relié d'un côté aux moyens 12 de traitement d'images et, de l'autre aux moyens 13 pour étirer le tissu. L'asservissement est soit assuré en continu, soit en discontinu, par exemple, à intervalle de temps, notamment régulièrement espacé.

Naturellement, d'autres mises en oeuvre de la présente invention, à la portée de l'homme de l'art, auraient pu être envisagées sans pour autant sortir du cadre de la pésente demande.

## Revendications

1. Procédé de contrôle de l'emplacement respectif de motifs 3 disposés selon une pseudo-période δ₀ pré-établie sur une nappe (1) de matériau, tel que, notamment, du tissu, du papier ou autre, selon lequel :
- on prévoit un champ d'investigation (4) incluant au moins deux motifs (3) complets,
- on prévoit une fenêtre de référence (5) incluse dans ledit champ d'investigation (4),
- on détecte, à l'intérieur dudit champ d'investigation (4) une fenêtre (6) optiquement identique à ladite fenêtre de référence (5) pour déterminer une pseudo-période à réelle.

2. Procédé selon la revendication 1, caractérisé par le fait que pour détecter ladite fenêtre optiquement identique (6) :
- on prévoit une plage de recherche (7) au voisinage de la pseudo-période et/ou d'un multiple de la pseudo-période δ₀,
- on prévoit une fenêtre de recherche (8) de dimension identique à la fenêtre de référence (5),
- on déplace pas à pas la fenêtre de recherche (8) dans le plan de la plage de recherche (7) et on compare, à chaque pas, l'image de ladite fenêtre de référence (5) et de ladite fenêtre de recherche (8),
- on repère un emplacement de ladite fenêtre de recherche pour lequel il existe une coincidence entre l'image de ladite fenêtre de recherche (8) et celle de la fenêtre de référence (5).

3. Procédé selon la revendication 2, caractérisé par le fait que pour comparer l'image de la fenêtre de recherche (8) et celle de la fenêtre de référence (5) :
- on réalise un découpage virtuel en sous-fenêtre de chacune des deux fenêtres,
- on mesure la luminosité de chacune des sous-fenêtres des deux fenêtres,
- on compare deux à deux, pour chaque sous-fenêtre correspondante des deux fenêtres, la luminosité,
- on additionne entre elles, pour chaque couple de sous-fenêtres, une valeur absolue de la différence de luminosité afin d'obtenir un niveau de différence.

4. Procédé selon la revendication 3, caractérisé par le fait que pour repérér l'emplacement pour lequel il existe une coïncidence entre l'image de ladite fenêtre de recherche (8) et celle de la fenêtre de référence (5), on détermine la fenêtre de recherche pour laquelle ledit niveau de différence est minimum.

5. Procédé selon la revendication 3, caractérisé par le fait que les sous-fenêtres des fenêtres de référence et de recherche sont constituées des pixels d'une caméra.

6. Procédé selon la revendication 5, caractérisé par le fait que pour connaître la luminosité de chacune des sous-fenêtres, on mesure le niveau de gris et/ou les niveaux de couleurs des pixels correspondants.

7. Procédé selon la revendication 1, caractérisé par le fait que l'on calcule et l'on affiche la pseudo-période réelle δ.

8. Application du procédé, selon l'une quelconque des revendications 1 à 7, dans lequel on contrôle l'emplacement respectif de motifs (3) disposés selon une pseudo-période δ₀ pré-établie sur une nappe de matériau (1) tel que, notamment, du tissu, dentelle ou autre, au suivi du droit dessin des motifs (3) disposés sur ladite nappe (1) par mesures successives de l'emplacement respectif desdits motifs (3).

9. Application du procédé selon l'une quelconque des revendications 1 à 7, dans lequel on contrôle l'emplacement respectif de motifs (3) disposés selon une pseudo-période δ₀ pré-établie sur une nappe de matériau (1) tel que, notamment, du tissu, dentelle ou autre, à une technique de préformage de ladite nappe avec asservissement du positionnement desdits motifs (3) selon les résultats dudit contrôle.

10. Application du procédé, selon l'une quelconque des revendications 1 à 7, dans lequel on contrôle l'emplacement respectif de motifs (3) disposés selon une pseudo-période δ₀ pré-établie sur une nappe de matériau (1) tel que notamment du tissu, dentelle, tricot ou autre à la régulation de la tension de ladite nappe à la sortie de machines à tisser, à fabriquer la dentelle, à tricoter ou équivalent, de manière à produire une nappe présentant des motifs répartis régulièrement.

11. Dispositif pour la mise en oeuvre du procédé, selon l'une quelconque des revendications 1 à 7, dans lequel on contrôle l'emplacement respectif de motifs (3) disposés selon une pseudo-période δ₀ pré-établie sur une nappe de matériau (1) tel que notamment, du tissu, du papier ou autre, notamment destiné au préformage de tissu présentant des motifs, caractérisé par le fait qu'il est constitué d'une caméra (11) et de moyens de traitement d'images (12).
